(19) Europäisches Patentamt
European Patent Office
Office européen des brevets

(11) Publication number: **0 280 503 B1**

(12) **EUROPEAN PATENT SPECIFICATION**

(45) Date of publication of patent specification: **07.04.93**

(51) Int. Cl.$^5$: **A61K 9/50**

(21) Application number: **88301512.5**

(22) Date of filing: **23.02.88**

(54) **Multivesicular liposomes having a biologically active substance encapsulated therein in the presence of a hydrochloride.**

(30) Priority: **23.02.87 GB 8704171**

(43) Date of publication of application:
**31.08.88 Bulletin 88/35**

(45) Publication of the grant of the patent:
**07.04.93 Bulletin 93/14**

(84) Designated Contracting States:
**AT BE CH DE ES FR GB GR IT LI LU NL SE**

(56) References cited:
**FR-A- 2 399 242**

**BIOCHEMICA ET BIOPHYSICA ACTA, vol. 728, 1983, pages 339-348; S. Kim et al.: "Preparation of multivesicular liposomes"**

(73) Proprietor: **RESEARCH DEVELOPMENT FOUNDATION**
**402 North Division Street**
**Carson City Nevada 89703(US)**

(72) Inventor: **Howell, Stephen Barnard**
**13612 Nogales Drive**
**Del Mar California 92014(US)**
Inventor: **Kim, Sinil**
**4016 Camino Calma**
**San Diego California 92122(US)**

(74) Representative: **Wilkinson, Stephen John et al**
**Stevens, Hewlett & Perkins 1 St. Augustine's Place**
**Bristol BS1 4UD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.5x/3.0.1)

## Description

### Field of the Invention

The invention relates to synthetic multivesicular lipid vesicles or liposomes encapsulating biologically active substances and processes for their manufacture.

### Background of the Invention

Multivesicular liposomes are one of the three main types of liposomes, first made by Kim, et. al. (1983, Biochim. Biophys. Acta 782, 339-348), and are uniquely different from the unilamellar (Huang, 1969, Biochemistry 8, 334-352; Kim, et. al. 1981, Biochim. Biophys. Acta 646, 1-10) and multilamellar (Bangham, et. al. 1965, J. Mol. Bio. 13, 238-252), liposomes in that there are multiple non-concentric aqueous chambers within. The prior art describes a number of techniques for producing liposomes, but all of these techniques relate to the production of non-multivesicular liposomes; for example, U.S. Patent No. 4,522,803 to Lenk; 4,310,506 to Baldeschwieler; 4,235,871 to Papahadjopoulos; 4,224,179 to Schneider; 4,078,052 to Papahadjopoulos; 4,394,372 to Taylor; 4,308,166 to Marchetti; 4,485,054 to Mezei; and 4,508,703 to Redziniak, all describe non-multivesicular vesicles. For a comprehensive review of various methods of liposome preparation, refer to Szoka, et. al., 1980, Ann. Rev. Biophys. Bioeng. 9:467-508.

The method of Kim, et. al. (1983, Biochim. Biophys. Acta 782,339-348) is the only report that describes multivesicular liposomes, but the encapsulation efficiency of some of the small molecules such as cytosine arabinoside was relatively low, and the leakage rate of encapsulated molecules in biological fluid was high.

Optimal treatment with many drugs requires maintenance of a drug level for a prolonged period of time. For example, optimal anti-cancer treatment with cell cycle-specific antimetabolites requires maintenance of a cytotoxic drug level for a prolonged period of time. Cytosine arabinoside is a highly scheduled-dependent anti-cancer drug. Because this drug kills cells only when they are making DNA, a prolonged exposure at therapeutic concentration of the drug is required for optimal cell kill. Unfortunately, the half-life of cytosine arabinoside after an intravenous (IV) or subcutaneous (SC) dose is very short. To achieve optimal cancer cell kill with a cell cycle phase-specific drug like cytosine arabinoside, two major requirements need to be met: first, the cancer must be exposed to a high concentration of the drug without doing irreversible harm to the host; and second, the tumor must be exposed for a prolonged period of time so that all or most of the cancer cells have attempted to synthesize DNA in the presence of cytosine arabinoside.

Prior to the present invention, the only way of achieving a prolonged plasma cell is through continuous IV or SC infusion, both of which are inconvenient and costly. Therefore, an acceptable slow-release in depot preparation of these drugs is needed. In the past, investigators have attempted to achieve this by chemical modification of the drug molecule to retard metabolism or covalent attachment of a hydrophobic moiety to retard solubilization. Such manipulations have resulted in new toxic effects, Finkelstein et. al., Cancer Treat Rep 63:1331-1333, 1979, or unacceptable pharmacokeinetic or formulation problems, Ho et. al., Cancer Res. 37:1640-1643, 1977.

Accordingly, a slow release depot preparation which provides a prolonged and sustained exposure at therapeutic concentration of a biologically-active substance is needed. The present invention is directed to such a preparation.

### Summary of the Invention

The present invention provides a multivesicular liposome containing a biologically active substance encapsulated in the presence of a hydrochloride which provides a prolonged and sustained exposure at therapeutic concentration of the biologically active substance for optimal results. The present invention also provides methods of making such multivesicular liposomes.

The multivesicular liposomes have high encapsulation efficiency, low leakage rate of the encapsulated substance, well defined, reproducible size distribution, spherical shape, adjustable average size that can be easily increased or decreased, adjustable internal chamber size and number.

The process for producing the multivesicular lipid vesicles or liposomes comprises dissolving in one or more organic solvents a lipid component containing at least one neutral lipid and at least one amphipathic lipid with one or more net negative charges, adding into the lipid component an immiscible first aqueous component containing a hydrochloride and one or more biologically active substances to be encapsulated, forming a water and oil emulsion from the two immiscible components, transferring and immersing the water and oil emulsion into a second immiscible aqueous component, dispersing the water and oil emulsion to form solvent spherules containing in them multiple droplets of the first aqueous component, and evaporating the organic solvents from the solvent spherules to form the multivesicular liposomes. The use of hydrochlorides, such as hydrochloric acid, is essential for high encapsulation efficiency and for slow leakage rate of encapsulated molecules in biological fluids and in vivo. When the hydrochloride is acidic, it is also essential to use a neutralizing agent of low ionic strength to prevent solvent spherules from sticking to each other.

Accordingly, it is an object of the present invention to provide a slow release depot preparation which provides a prolonged and sustained exposure of a biologically active substance at a therapeutic concentration.

It is a further object of the present invention to provide a method of preparing such a depot preparation.

A further object of the present invention is the provision of a multivesicular liposome having a biologically active substance encapsulated therein and having a prolonged and sustained exposure at a therapeutic concentration.

It is a further object of the present invention is the provision of a multivesicular liposome containing a biologically active substance encapsulated within it in the presence of a hydrochloride which provides a prolonged exposure at therapeutic concentration of the biological active substance for optimal results.

A further object of the present invention is the provision of a method of preparing such a multivesicular liposome.

Other and further objects, features, and advantages of the invention are inherent therein and appear throughout the specification and claims.

## Description of Preferred Embodiments

The term "multivesicular liposomes" as used throughout the specification and claims means man-made, microscopic lipid-vesicles consisting of lipid bilayer membranes, enclosing multiple non-concentric aqueous chambers. In contrast, unilamellar liposomes have a single aqueous chamber; and multilamellar liposomes have multiple "onion-skin" type of concentric membranes, in between which are shell-like concentric aqueous compartments.

The term "solvent spherule" as used throughout the specification and claims means a microscopic spheroid droplet of organic solvent, within which is multiple smaller droplets of aqueous solution. The solvent spherules are suspended and totally immersed in a second aqueous solution.

The term "neutral lipid" means oil or fats that have no membrane-forming capability by themselves and lack hydrophilic "head" group.

The term "amphipathic lipids" means those molecules that have a hydrophilic "head" group and hydrophobic "tail" group and have membrane-forming capability.

The term "ionic strength" is defined as: Ionic strength = $1/2\ (M_1 Z_1^2 + M_2 Z_2^2 + M_3 Z_3^2 + ...)$ where $M_1 M_2 M_3$ ... represent the molar concentrations of various ions in the solution and $Z_1 Z_2 Z_3$ ... are their respective charges.

The term "low ionic strength" is ionic strength less than approximately 0.05, and preferably less than 0.01.

Briefly, "water-in-lipid" emulsion is first made by dissolving amphipathic lipids in a volatile organic solvent for the lipid component, adding to the lipid component an immiscible first aqueous component, the substance to be encapsulated and hydrochloride, and then emulsifying the mixture mechanically. In the emulsion, the water droplets suspend in the organic solvent will form the internal aqueous chambers, and the monolayer of amphipathic lipids lining the aqueous chambers will become one leaflet of the bilayer membrane in the final product. The whole emulsion is then immersed in the second aqueous component containing one or more nonionic osmotic agents and an acid-neutralizing agent of low ionic strength and then agitated either mechanically, by ultrasonic energy, nozzle atomizations or combinations thereof to form solvent spherules suspended in the second aqueous component. The solvent spherules contain multiple aqueous droplets with the substance to be encapsulated dissolved in them. The volatile organic solvent is evaporated from the spherules by passing a stream of gas over the suspension. When the solvent is completely evaporated, the spherules convert into multivesicular liposomes. The average size of the liposome particles and number of aqueous chambers within them are determined by the type, intensity and duration of the method selected. Representative gases satisfactory for use include nitrogen, helium, argon, oxygen, hydrogen and carbon dioxide.

The use of a hydrochloride is essential for high encapsulation efficiency and for a slow leakage rate of encapsulated molecules in biological fluids and in vivo. When the hydrochloride is acidic, it is also essential to use a neutralizing agent of low ionic strength to prevent the solvent spherules from sticking to each other.

Hydrochloric acid is preferred but other hydrochlorides which are satisfactory include lysine hydrochloride, histidine hydrochloride and combinations thereof. The amounts of both the hydrochloride and the neutralizing agent used can range from about 0.1mM to about 0.5M concentrations and preferably from about 10mM to about 200mM concentration.

Many different types of volatile hydrophobic solvents such as ethers, hydrocarbons, halogenated hydrocarbons, or Freons$^{R}$ may be used as the lipid-phase solvent. For example, diethyl ether, isopropyl and other ethers, chloroform, tetrahydrofuran, halogenated ethers, esters and combinations thereof are satisfactory.

In order to prevent the solvent spherules from sticking to each other and to the vessel wall, at least 1 percent molar ratio of an amphipathic lipid with a net negative charge needs to be included in the spherules, the suspending aqueous solution needs to have a very low ionic strength, and, when the hydrochloride is an acid, a neutralizing agent of low ionic strength is needed to absorb the hydrochloride, such as free-base lysine, free-base histidine or a combination thereof; otherwise, the solvent spherules coalesce to form a messy scum. One or more nonionic osmotic agents are needed in the suspending aqueous solution to keep the osmotic pressure within and without the liposomes balanced. Various types of lipids can be used to make the multivesicular liposomes, and the only two requirements are that one neutral lipid and one amphipathic lipid with a net negative charge be included. Examples of neutral lipids are triolein, trioctanoin, vegetable oil such as soybean oil, lard, beef fat, tocopherol, and combinations thereof. Examples of amphipathic lipids are a phospholipid or an admixture of phospholipids selected from the group consisting of phosphatidylcholine, cardiolipin, phosphatidylethanolamine, sphingomyelin, lysophosphatidylcholine, phosphatidylserine, phosphatidylinositol, phosphatidylglycerol, and phosphatidic acid. These phospholipids may be admixed with other phospholipids such as cholesterol or stearylamine. Further, lipophilic biologically active material may be admixed with the lipid component. Examples of amphipathic lipids with net negative charge are cardiolipin, the phosphatidylserines, phosphatidylglycerols, and phosphatidic acids.

The second aqueous component is an aqueous solution containing solutes such as carbohydrates including glucose, sucrose, lactose, and amino acids such as lysine, free-base histidine and combinations thereof.

Many and varied biological substances can be incorporated by encapsulation within the multivesicular liposomes. These include drugs, and other kinds of materials, such as DNA, RNA, proteins of various types, protein hormones produced by recombinant DNA technology effective in humans, hematopoietic growth factors, monokines, lymphokines, tumor necrosis factor, inhibin, tumor growth factor alpha and beta, Mullerian inhibitory substance, nerve growth factor, fibroblast growth factor, platelet-derived growth factor, pituitary and hypophyseal hormones including LH and other releasing hormones, calcitonin, proteins that serve as immunogens for vaccination, and DNA and RNA sequences.

The following Table 1 includes a list of representative biologically active substances which can be encapsulated in multivesicular liposomes in the presence of a hydrochloride and which are effective in humans.

Table 1

| Antiasthma | Antiarrhythimic | Tranquilizers |
|---|---|---|
| metaproterenol | propanolol | chlorpromazine |
| aminophylline | atenolol | benzodiazepine |
| theophylline | verapamil | butyrophenones |
| terbutaline | captopril | hydroxyzines |
| Tegretol® | isosorbide | meprobamate |
| ephedrine | | phenothiazines |
| isoproterenol | | reserpine |
| adrenalin | | thioxanthines |
| norepinephrine | | |

| Cardiac glycosides | Hormones | Steroids |
|---|---|---|
| digitalis | antidiuretic | prednisone |
| digitoxin | corticosteroids | triamcinolone |
| lanatoside C | testosterone | hydrocortisone |
| digoxin | estrogen | dexamethasone |
| | thyroid | betamethosone |
| | growth | prednisolone |
| | ACTH | |
| | progesterone | |
| | gonadotropin | |
| | mineralocorticoid | |
| | LH | |
| | LHRH | |
| | FSH | |
| | calcitonin | |

| Antihypertensives | Antidiabetic | Antihistamines |
|---|---|---|
| apresoline | Diabenese® | pyribenzamine |
| atenolol | insulin | chlorpheniramine |
| | | diphenhydramine |

| Antiparasitic | Anticancer | Sedatives & Analgesic |
|---|---|---|
| praziquantel | azathioprine | morphine |
| metronidazole | bleomycin | dilaudid |
| pentamidine | cyclophosphamide | codeine |
| | adriamycin | codeine-like synthetics |
| | daunorubicin | demerol |
| | vincristine | oxymorphone |
| | methotrexate | phenobarbital |
| | 6-TG | barbiturates |
| | 6-MP | |
| | vinblastine | |
| | VP-16 | |
| | VM-26 | |
| | cisplatin | |
| | FU | |

| Antibiotic | Immunotherapies | Vaccines |
|---|---|---|
| penicillin | interferon | influenza |
| tetracycline | interleukin-2 | respiratory syncytial virus |
| erythromycin | monoclonal antibodies | Hemophilus influenza vaccine |
| cephalothin | gammaglobulin | |
| imipenem | | |
| cefofaxime | **Antifungal** | **Antiviral** |
| carbenicillin | | |
| vancomycin | amphotericin B | acyclovir and derivatives |
| gentamycin | myconazole | Winthrop-51711® |
| tobramycin | muramyl dipeptide | ribavirin |
| piperacillin | clotrimazole | rimantadine/amantadine |
| moxalactam | | azidothymidine & derivatives |
| amoxicillin | **Antihypotension** | adenine arabinoside |
| ampicillin | | amidine-type protease inhibitors |
| cefazolin | dopamine | |
| cefadroxil | dextroamphetamine | **Other** |
| cefoxitin | | cell surface receptor blockers |
| other aminoglycosides | | |

**Proteins and Glycoproteins**
- lymphokines
  - interleukins - 1, 2, 3, 4, 5, and 6
- cytokines
  - GM-CSF
  - M-CSF
  - G-CSF
- tumor necrosis factor
- inhibin
- tumor growth factor
- mullerian inhibitors substance
- nerve growth factor
- fibroblast growth factor
- platelet derived growth factor
- coagulation factors (e.g. VIII, IX, VII)
- insulin
- tissue plaminogen activator
- histocompatibility antigen
- oncogene products
- myelin basic protein
- collagen
- fibronectin
- laminin
- other proteins made by recombinant DNA technology

**Nucleic Acids & Analogs**
- DNA
- RNA
- methylphosphonates and analogs

The dosage range appropriate for human use include the range of 1-6000 mg/m to body surface area. The reason that this range is so large is that for some applications, such as subcutaneous administration, the dose required may be quite small, but for other applications, such as intraperitoneal administration, the dose desired to be used may be absolutely enormous. While doses outside the foregoing dose range may be given, this range encompasses the breadth of use for practically all the biologically active substances. The biologically active substance may be a hydrophilic biologically active material. However, lipophilic biologically active materials may be admixed with the lipid component.

The multivesicular liposomes may be administered by any desired route; for example, intrathecal, intraperitoneal, subcutaneous, intravenous, intralymphatic, oral and submucosal, under many different kinds of epithelia including the bronchialar epithelia, the gastrointestinal epithelia, the urogenital epithelia, and various mucous membranes of the body, and intramuscular.

The following examples represent presently preferred methods of preparing these multivesicular liposomes encapsulating biologically active substances.

**EXAMPLE 1**

Step 1) In a clean 4 ml (one-dram) glass vial (1.4 cm diameter X 4.5 cm height in external dimensions), 9.3 µmoles of dioleoyl lecithin, 2.1 µmoles of dipalmitoyl phosphatidylglycerol, 15 µmoles of cholesterol, 1.8 µmoles of triolein and one ml of chloroform were placed (the lipid phase).

Step 2) One ml of aqueous phase, cytosine arabinoside (20 mg./ml) dissolved in 0.136 N hydrochloric acid solution, is added into the above 4 ml (one-dram) vial containing lipid phase.

Step 3) For making the water-in-oil emulsion, the vial is sealed and attached to the head of a vortex shaker and shaken at maximum speed for 6 minutes.

Step 4) For making the chloroform spherules suspended in water, half of the emulsion is then each squirted rapidly through a narrow tip Pasteur pipette into 4 ml (one-dram) vials, each containing 4 percent dextrose in water and 40 mM lysine, free base, and then shaken on the vortex shaker for 3 seconds at half speed to form the chloroform spherules.

Step 5) The chloroform spherule suspensions in the two vials are poured into the bottom of a 250 ml Erlenmeyer flask containing 5 ml of water, glucose (3.5 g/100 ml), and free-base lysine (40 mM) and a stream of nitrogen gas at 7 l/minute is flushed through the flask to slowly evaporate chloroform over 10 - 15 minutes at 37° C. The liposomes are then isolated by centrifugation at 600 X g for 5 minutes.

The average volume-adjusted size of the resulting liposomes (± standard deviation of the distribution) was 19.4 ± 6.5 µm. Percentage of capture was 59 ± 7 percent and capture volume was 36 ± 4 µl/mg of total lipids used. The addition of hydrochloric acid had marked influence on the rate of cytosine arabinoside leakage from the multivesicular liposomes incubated in human plasma. Half of the drug leaked out in 12 days when hydrochloric acid is added in the example above, whereas half of the drug leaked out in only 12 hours when the hydrochloric acid was omitted in step 2 above. When tested in animals, the addition of hydrochloric again had marked influence on the rate of cytosine arabinoside release; the drug stayed within mouse peritoneal cavity much longer when hydrochloric acid is added during manufacture.

The following example illustrates a scale up of the procedure for making large batches of multivesicular liposomes.

EXAMPLE 2

Step 1) In a stainless-steel homogenizer (Omni-Mixer® 17150, Sorval Co., Newtown, CT) add 186 µmoles of dioleoyl lecithin, 42 µmoles of dipalmitoyl phosphatidylglycerol, 300 µmoles of cholesterol, 36 µmoles of triolein and 20 ml of chloroform were placed (the lipid phase).

Step 2) Twenty ml of aqueous phase, cytosine arabinoside (20 mg/ml) dissolved in 0.136 N hydrochloric acid solution is added into the above stainless-steel mixer containing lipid phase while swirling.

Step 3) For making the water-in-oil emulsion, the homogenizer is sealed and run at "7" setting for 3 minutes.

Step 4) For making the chloroform spherules suspended in water, half of the emulsion is then each poured (while swirling) into two other stainless-steel homogenizer vessels, each containing 200 ml of 4 percent dextrose in water and 40 mM lysine, free base, and then homogenized on the Omni-Mixer® for 3 seconds at "1" setting.

Step 5) The chloroform spherule suspensions in each homogenizer vessels are poured into a flat-bottomed, rectangular steel container, 8 in X 12 in the bottom dimension and a stream of nitrogen gas or air at 14 l/minute was flushed through the flask to slowly evaporate chloroform over 10 - 15 minutes. The liposomes are then isolated by centrifugation at 600 X g for 5 minutes.

The following example illustrates methods of making smaller or larger liposomes.

EXAMPLE 3

To make liposomes smaller than that in Example 1 or 2, the mechanical strength or duration of shaking or homogenization in Step 4 of Example 1 or 2 was increased. To make liposomes larger, the mechanical strength or duration of shaking or homogenization in Step 4 of Example 1 or 2 was decreased.

The following Example 4 illustrates representative methods of making liposomes of various lipid compositions and incorporating various materials into liposomes.

EXAMPLE 4

In Step 1) of Example 1 or 2, other amphipathic lipids such as phosphatidyl cholines (PC), cardiolipin (CL), dimyristoyl phosphatidylglycerol (DMPG), phosphatidyl ethanolamines (PE), phaphatidyl serines (PS), dimyristoyl phosphatidic acid (DMPA) in various combinations can be used with similar results. For example, PC/C/CL/TO in 4.5/4.5/1/1 molar ration; DOPC/C/PS/TO in 4.5/4.5/1/1 molar ratio; PC/C/DPPG/TC in 5/4/1/1 molar ratio; I PC/C/PG/TC in 5/4/1/1 molar ratio; PE/C/CL/TO in 4.5/4.5/1/1 molar ratio; PC/C/DMPA/TO in 4.5/4.5/1/1 molar ratio can all be used. To incorporate other biologically active materials, simply substitute the cytosine arabinoside with a desired material or combination of materials of Table 1 in Step 2 of Example 1 or 2. All of these provide multivesicular liposomes which are effective in humans and provide prolonged exposure of the biologically-active substance at therapeutic concentration.

Thus, the present invention provides "depot" preparations of wide application and uses in which biologically active substances are encapsulated in relatively large amounts and provide prolonged exposure at therapeutic concentrations of these substances for optimal results which avoid high peaking of dosage, which could be toxic.

The present invention, therefore, is well suited and adapted to attain the ends and objects and has the advantages and features mentioned as well as others inherent therein.

**Claims**

**1.** A process for producing multivesicular lipid vesicles or liposomes comprising the steps of:

(a) dissolving a lipid component in one or more organic solvents wherein the said lipid component contains at least one neutral lipid and at least one amphipathic lipid with at least one net negative charge;

(b) adding into the said lipid component an immiscible first aqueous component containing a hydrochloride and one or more biologically active substances to be encapsulated;

(c) forming a water-in-oil emulsion from the two immiscible components;

(d) transferring and immersing the water-in-oil emulsion into a second immiscible aqueous component;

(e) dispersing the water-in-oil emulsion to form solvent spherules containing multiple droplets of the first aqueous component therein; and

(f) evaporating the organic solvents from the solvent spherules to form the multivesicular liposomes.

**2.** The process according to Claim 1 wherein the lipid component is selected from the group consisting of a phospholipid and an admixture of phospholipids.

**3.** The process according to Claim 2 wherein the phospholipids are selected from the group consisting of phosphatidylcholine, cardiolipin, phosphatidylethanolamine, sphingomyelin, lysophosphatidylcholine, phosphatidylserine, phosphatidylinositol, phosphatidylglycerol, and phosphatidic acid.

**4.** The process according to Claim 2 wherein
at least one of the phospholipids is selected from the group with at least one net negative charge.

**5.** The process according to Claim 2 wherein
the phospholipid is provided in admixture with cholesterol.

**6.** The process according to Claim 2 wherein
the phospholipid is provided in admixture with stearylamine.

**7.** The process according to Claim 1 wherein
a lipophilic biologically active material is provided in admixture with the lipid component.

**8.** The process according to Claim 1 wherein
the neutral lipid is selected from the group consisting of triolein, trioctanoin, vegetable oil, lard, beef fat, tocopherol, and combinations thereof.

**9.** The process according to Claim 1 wherein,
the organic solvent is selected from the group consisting of diethyl ether, isopropyl ether, chloroform, tetrahydrofuran, ethers, hydrocarbons, halogenated hydrocarbons, halogenated ethers, esters, and combinations thereof.

**10.** The process according to Claim 1 wherein the hydrochloride is selected from the group consisting of hydrochloric acid, lysine hydrochloride, histadine hydrochloride, and combinations thereof.

**11.** The process according to Claim 1 where the substance is a hydrophilic biologically active material.

**12.** The process according to Claim 1 wherein
the emulsification of the two said components is carried out using a method selected from the group consisting of mechanical agitation, ultrasonic energy, and nozzle atomization.

**13.** The process according to Claim 12 wherein
the liposome's average size and number of the aqueous chambers within them are determined by the type, intensity, and duration of the method selected.

**14.** The process according to Claim 1 wherein
the hydrochloride is acidic, and
the second aqueous component contains at least one neutralizing agent.

**15.** The process according to Claim 14 wherein
the neutralizing agent is selected from the group consisting of free-base lysine and free base histidine and a combination thereof.

**16.** The process according to Claim 1 wherein
the second aqueous component is of low ionic strength.

**17.** The process according to Claim 16 wherein the second aqueous component is an aqueous solution containing solutes selected from the group consisting of carbohydrates and amino acids.

**18.** The process according to Claim 16 wherein the second aqueous component is an aqueous solution containing solutes selected from the group consisting of glucose, sucrose, lactose, free-base lysine, free-base histidine, and combinations thereof.

**19.** The process according to Claim 1 wherein the formation of the solvent spherules is carried out using methods selected from the group consisting of mechanical agitation, ultrasonic energy, nozzle atomization, and combinations thereof.

**20.** The process according to Claim 19 wherein the liposome's average size is determined by the type, intensity, and duration of the energy used.

**21.** The process according to Claim 1 wherein the evaporation of the organic solvent is provided by passing gas over the second aqueous components.

**22.** The process of Claim 1 where, the biologically active substance to be encapsulated is selected from the group consisting of the compositions of Table 1 and combinations thereof.

**Patentansprüche**

**1.** Verfahren zur Herstellung von multivesikularen Lipidvesikeln oder Liposomen, umfassend die Schritte
(a) Lösen eines Lipidbestandteils in einem oder mehreren organischen Lösungsmitteln, wobei der Lipidbestandteil mindestens ein neutrales Lipid und mindestens ein amphipathisches Lipid mit netto mindestens einer negativen Ladung enthält;
(b) Zufügen eines unmischbaren ersten wäßrigen Bestandteils, enthaltend ein Hydrochlorid und ein oder mehrere biologisch wirksame Substanzen, die verkapselt werden sollen, zu diesem Lipidbestandteil;
(c) Bilden einer Wasser-in-Öl-Emulsion aus den beiden unmischbaren Bestandteilen;

(d) Überführen und Einbringen der Wasser-in-Öl-Emulsion in einen zweiten unmischbaren wäßrigen Bestandteil;
(e) Dispergieren der Wasser-in-Öl-Emulsion, damit sich Lösungsmittelkügelchen bilden, die eine Vielzahl von Tröpfchen des ersten wäßrigen Bestandteils enthalten; und
(f) Verdampfen der organischen Lösungsmittel von den Lösungsmittelkügelchen, damit sich multivesikulare Liposome ausbilden.

**2.** Verfahren nach Anspruch 1, wobei der Lipidbestandteil ausgewählt ist aus der Gruppe von Phospholipiden und einem Gemisch von Phospholipiden.

**3.** Verfahren nach Anspruch 2, wobei die Phospholipide ausgewählt sind von der Gruppe Phosphatidylcholin, Cardiolipin, Phosphatidylethanolamin, Sphingomyelin, Lysophosphatidylcholin, Phosphatidylserin, Phosphatidylinositol, Phosphatidylglycerol und Phosphatidsäure.

**4.** Verfahren nach Anspruch 2, wobei mindestens eines der Phospholipide ausgewählt ist aus der Gruppe mit netto mindestens einer negativen Ladung.

**5.** Verfahren nach Anspruch 2, wobei das Phospholipid im Gemisch mit Cholesterin vorliegt.

**6.** Verfahren nach Anspruch 2, wobei das Phospholipid im Gemisch mit Stearylamin vorliegt.

**7.** Verfahren nach Anspruch 1, wobei das lipophile biologisch wirksame Material im Gemisch mit dem Lipidbestandteil vorliegt.

**8.** Verfahren nach Anspruch 1, wobei das neutrale Lipid ausgewählt ist aus der Gruppe Triolein, Trioctanoin, Pflanzenöl, Schweinefett, Rinderfett, Tocopherol und Gemischen davon.

**9.** Verfahren nach Anspruch 1, wobei das organische Lösungsmittel ausgewählt ist aus der Gruppe Diethylether, Isopropylether, Chloroform, Tetrahydrofuran, Ether, Kohlenwasserstoffe, halogenierte Kohlenwasserstoffe, halogenierte Ether, Ester und Gemische davon.

**10.** Verfahren nach Anspruch 1, wobei das Hydrochlorid ausgewählt ist aus der Gruppe Salzsäure, Lysinhydrochlorid, Histidinhydrochlorid und Gemischen davon.

**11.** Verfahren nach Anspruch 1, wobei die Substanz ein hydrophiles biologisch wirksames Material ist.

**12.** Verfahren nach Anspruch 1, wobei das Emulsieren der zwei Bestandteile unter Verwendung eines Verfahrens durchgeführt wird, das ausgewählt ist aus der Gruppe mechanisches Rühren, Ultraschallenergie, und Düsenversprühung.

**13.** Verfahren nach Anspruch 12, wobei die mittlere Größe der Liposomen und die Zahl der wäßrigen Kammern darin über die Art, die Intensität und die Dauer des gewählten Verfahrens bestimmt werden.

**14.** Verfahren nach Anspruch 1, wobei das Hydrochlorid sauer ist und der zweite wäßrige Bestandteil mindestens ein neutralisierendes Mittel enthält.

**15.** Verfahren nach Anspruch 14, wobei das neutralisierende Mittel ausgewählt ist aus der Gruppe Lysin als freie Base, Histidin als freie Base und Gemische davon.

**16.** Verfahren nach Anspruch 1, wobei der zweite wäßrige Bestandteil eine niedrige Ionenstärke aufweist.

**17.** Verfahren nach Anspruch 16, wobei der zweite wäßrige Bestandteil eine wäßrige Lösung ist, die gelöste Stoffe, ausgewählt aus der Gruppe der Kohlenhydrate und Aminosäuren, enthält.

**18.** Verfahren nach Anspruch 16, wobei der zweite wäßrige Bestandteil eine wäßrige Lösung ist, die gelöste Stoffe enthält, die ausgewählt sind aus der Gruppe Glucose, Saccharose, Lactose, Lysin als freie Base, Histidin als freie Base und Gemischen davon.

19. Verfahren nach Anspruch 1, wobei die Bildung der Lösungsmittelkügelchen unter Verwendung eines Verfahrens durchgeführt wird, das aus der Gruppe bestehend aus mechanischem Rühren, Ultraschallenergie, Düsenversprühung und Kombinationen davon ausgewählt ist.

20. Verfahren nach Anspruch 19, wobei die mittlere Größe der Liposomen durch die Art, die Intensität und die Dauer der verwendeten Energie bestimmt wird.

21. Verfahren nach Anspruch 1, wobei die Verdampfung des organischen Lösungsmittels dadurch erfolgt, daß ein Gas über die zweiten wäßrigen Bestandteile geleitet wird.

22. Verfahren nach Anspruch 1, wobei die biologisch wirksame Substanz, die verkapselt werden soll aus der Gruppe bestehend aus den Zusammensetzungen von Tabelle I und Kombinationen davon ausgewählt wird.

## Revendications

1. Procédé pour la préparation de vésicules lipidiques ou liposomes multivésiculaires comprenant les étapes de :
   (a) dissolution d'un composant lipidique dans un ou plusieurs solvants organiques, ledit composant lipidique contenant au moins un lipide neutre et au moins un lipide amphipathe avec au moins une charge globale négative ;
   (b) addition audit composant lipidique d'un premier composant aqueux non miscible contenant un chlorhydrate et une ou plusieurs substances biologiquement actives à encapsuler ;
   (c) formation d'une émulsion eau-dans-huile à partir des deux composants non miscibles ;
   (d) transfert et immersion de l'émulsion eau-dans-huile dans un second composant aqueux non miscible.
   (e) dispersion de l'émulsion eau-dans-huile pour former des sphérules de solvant contenant plusieurs gouttelettes aqueuses renfermant le premier composant aqueux ; et
   (f) évaporation des solvants organiques des sphérules de solvant pour former les liposomes multivésiculaires.

2. Procédé selon la revendication 1 dans lequel le composant lipidique est choisi dans le groupe formé d'un phospholipide et d'un mélange de phospholipides.

3. Procédé selon la revendication 2 dans lequel les phospholipides sont sélectionnés dans le groupe consistant en phosphatidylcholine, cardiolipine, phosphatidyléthanolamine, sphingomyéline, lysophosphatidylcholine, phosphatidylsérine, phosphatidylinositol, phosphatidylglycérol et acide phosphatidique.

4. Procédé selon la revendication 2 dans lequel au moins un des phospholipides est sélectionné dans le groupe comprenant au moins une charge globale négative.

5. Procédé selon la revendication 2 dans lequel le phospholipide est mélangé au cholestérol.

6. Procédé selon la revendication 2 dans lequel le phospholipide est mélangé à la stéarylamine.

7. Procédé selon la revendication 1 dans lequel un matériau biologiquement actif lipophile est mélangé au composant lipidique.

8. Procédé selon la revendication 1 dans lequel le lipide neutre est sélectionné dans le groupe comprenant trioléine, trioctanoïne, huile végétale, lard, graisse de boeuf tocophérol et leurs combinaisons.

9. Procédé selon la revendication 1 dans lequel le solvant organique est sélectionné dans le groupe comprenant l'éther diéthylique, l'éther isopropylique, le chloroforme, le tétrahydrofuranne, des éthers, des hydrocarbures, des hydrocarbures halogénés, des éthers halogénés, des esters et leurs combinaisons.

10. Procédé selon la revendication 1 dans lequel le chlorhydrate est sélectionné dans le groupe comprenant l'acide chlorhydrique, le chlorhydrate de lysine, le chlorhydrate d'histidine et leurs combinaisons.

11. Procédé selon la revendication 1 dans lequel la substance est un matériau biologiquement actif hydrophile.

12. Procédé selon la revendication 1 dans lequel l'émulsification desdits deux composants est réalisée par une méthode sélectionnée dans le groupe comprenant l'agitation mécanique, l'énergie ultrasonore et la pulvérisation par gicleur.

13. Procédé selon la revendication 12 dans lequel la dimension moyenne des liposomes et le nombre de chambres aqueuses contenues dans les liposomes sont déterminés par le type, l'intensité et la durée de la méthode sélectionnée.

14. Procédé selon la revendication 1 dans lequel le chlorhydrate est acide, et le second composant aqueux contient au moins un agent neutralisant.

15. Procédé selon la revendication 14 dans lequel l'agent neutralisant est sélectionné dans le groupe composé de la lysine base libre et de l'histidine base libre et d'une de leurs combinaisons.

16. Procédé selon la revendication 1 dans lequel le second composant aqueux a une faible force ionique.

17. Procédé selon la revendication 16 dans lequel le second composant aqueux est une solution aqueuse contenant des solutés sélectionnés dans le groupe comprenant des hydrates de carbone et des amino acides.

18. Procédé selon la revendication 16 dans lequel le second composant aqueux est une solution aqueuse contenant des solutés sélectionnés dans le groupe comprenant le glucose, le saccharose, le lactose, la lysine base libre, l'histidine base libre, et leurs combinaisons.

19. Procédé selon la revendication 1 dans lequel la formation des sphérules de solvant est réalisée par une méthode sélectionnée dans le groupe comprenant l'agitation mécanique, l'énergie ultrasonore, la pulvérisation par gicleur et leurs combinaisons.

20. Procédé selon la revendication 19 dans lequel la dimension moyenne des liposomes est déterminée par le type, l'intensité et la durée de l'énergie utilisée.

21. Procédé selon la revendication 1 dans lequel l'évaporation du solvant organique est réalisée en faisant passer un gaz sur les seconds composants aqueux.

22. Procédé selon la revendication 1 dans lequel la substance biologique à encapsuler est sélectionnée dans le groupe constitué par des compositions du tableau 1 et leurs combinaisons.